# DEMANDE DE BREVET EUROPEEN

(11) **EP 2 181 720 A1**
(43) Date de publication de la demande: **05.05.2010**
(21) Numéro de dépôt: 09354047.4
(22) Date de dépôt: 03.11.2009
(51) Int. Cl.: A61L 9/20, B01D 53/00, F24F 3/00

(54) **Dispositif de décontamination d'air**

(30) Priorité: 04.11.2008 FR 0806142
(71) Demandeur: Techno-Alpes-Ing, 38760 Saint-Paul-de-Varces (FR)
(72) Inventeur: Archen, Jean-Marc, 38760 Saint Paul de Varces (FR); Archen, Frédéric, 38450 Saint Georges de Commiers (FR); Gerard, Eric, 38490 Granieu (FR)
(74) Mandataire: Dubreu, Sandrine

(57) **Abrégé**

Dispositif de décontamination d'air par photocatalyse qui comporte :
- une enceinte de photocatalyse (1) pourvue d'au moins un orifice d'entrée (3) et un orifice de sortie (4),
- au moins une source lumineuse (6) pour activer la photocatalyse,
- au moins un support (5) comportant un photocatalyseur, ladite source lumineuse (6) illuminant au moins une surface du support (5),
- des moyens de circulation de l'air (2) à décontaminer en provenance de l'orifice d'entrée (3) vers l'orifice de sortie (4) de ladite l'enceinte (1) et,
- un composé métallique solide choisi parmi un métal germicide, bactériostatique ou antifongique.

Le support (5) est poreux et pourvu d'une première couche interne comportant le composé métallique solide, d'au moins une seconde couche interne, les première et seconde couches internes étant disposées entre deux couches externes comportant le photocatalyseur. Un écoulement d'air léchant et traversant le support (5) est créé.

## Description

### Domaine technique de l'invention

L'invention concerne un dispositif de décontamination d'air par photocatalyse comportant :
- une enceinte de photocatalyse pourvue d'au moins un orifice d'entrée et un orifice de sortie,
- au moins une source lumineuse pour activer la photocatalyse,
- au moins un support comportant un photocatalyseur, ladite source lumineuse illuminant au moins une surface du support,
- des moyens de circulation de l'air à décontaminer en provenance de l'orifice d'entrée vers l'orifice de sortie de ladite l'enceinte et,
- un composé métallique solide choisi parmi un métal germicide, bactériostatique ou antifongique.

### État de la technique

L'air contient de nombreux contaminants tels que des composés organiques volatils (COV), des composés inorganiques et des microorganismes tels que des bactéries, virus ou moisissures. De façon courante, les photocatalyseurs sont utilisés pour purifier l'air par décomposition de ces contaminants en sous-produits faciles à éliminer tel que le dioxyde de carbone. La photocatalyse consiste à activer le catalyseur par irradiation, par exemple, sous rayonnement ultraviolet (UV), pour former des radicaux libres aptes à dégrader notamment les chaînes carbonées organiques, absorbées par le photocatalyseur. La vitesse de réaction de conversion des contaminants, basée sur des phénomènes d'absorption et de dégradation complexes, dépend de plusieurs facteurs notamment du temps de contact de l'air avec le photocatalyseur. Les photocatalyseurs les plus couramment utilisés sont, par exemple, les oxydes de terres rares, d'alcalino-terreux ou métalliques. Conventionnellement, les dispositifs de décontamination d'air sont pourvus d'un photocatalyseur fixé sur un support et d'un système de ventilation pour la mise en contact de l'air à décontaminer avec le photocatalyseur. Le support est généralement disposé perpendiculaire à l'écoulement de l'air, générant un mouvement d'air traversant le support afin d'augmenter le temps de contact entre l'air et le photocatalyseur. À titre d'exemple, la demande de brevet FR2914558 décrit un dispositif de traitement d'air par photocatalyse comportant une enceinte de photocatalyse, un moyen de ventilation et un support poreux contenant le photocatalyseur, entourant la source de photons. Par ailleurs, la source de photons est, préférentiellement, une lampe à rayonnement UV-C afin de conjuguer l'effet irradiant et germicide des UV-C. Néanmoins, la configuration perpendiculaire du support par rapport à l'écoulement d'air induit conjointement une résistance au flux qui crée une variation de pression entre l'entrée et la sortie du dispositif. Pour compenser cette différence de pression dans l'enceinte de photocatalyse, des opérations additionnelles de régulation de flux d'air sont nécessaires, engendrant des surcoûts de fonctionnement. Pour assurer un écoulement ordonné dit "laminaire" à travers le dispositif, il est alors nécessaire d'opposer le moins de résistance possible au flux d'air. Par ailleurs, les rayons UV-C, à courte longueur d'onde c'est-à-dire comprise entre 10nm et 280nm, sont très nocifs. Leur utilisation nécessite donc des précautions particulières.

D'autres dispositifs ont également été proposés. Notamment, le document US-B-6797127 décrit un dispositif et un procédé pour purifier l'air qui génère la formation d'oxygène actif. Le dispositif permet de décomposer des produits organiques par irradiation de l'oxygène de l'air en présence d'un photocatalyseur à base d'oxyde de titane supportant de fines particules de cuivre jouant le rôle d'électrode. Le photocatalyseur est déposé sur les parois de séparation de trois chambres de photocatalyse et constituent le support du photocatalyseur.

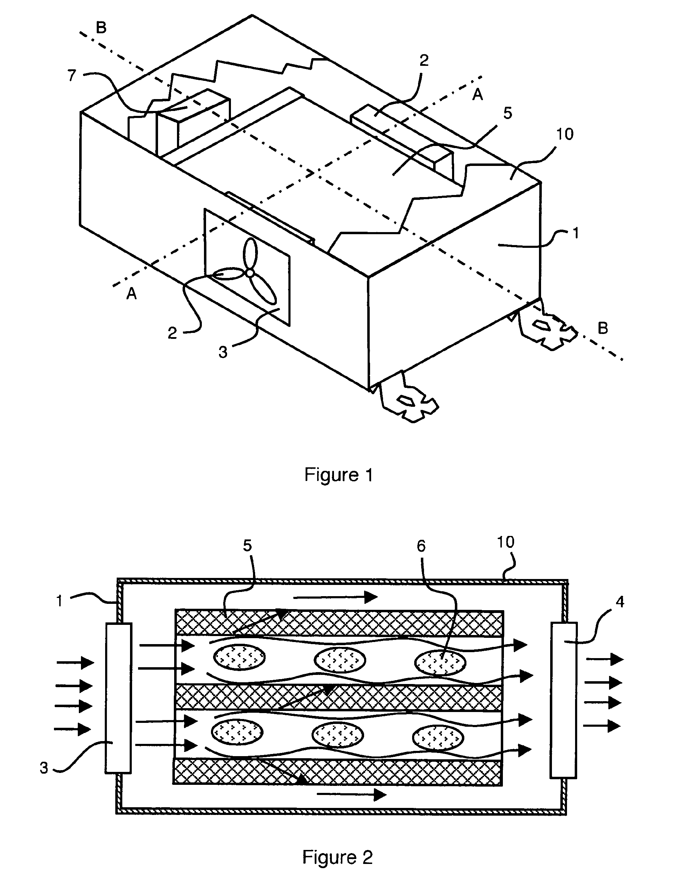

Le document EP-A-0978690 décrit un dispositif de décontamination d'air formé d'un photocatalyseur à base d'oxyde de titane sous forme d'une feuille poreuse ou percée de trous. Cette feuille, flexible et pliable, est constituée d'un substrat recouvert d'une couche de particules de photocatalyseur dispersées dans une résine de tétrafluoroéthylène (figure 1).

Le document EP-A-1843401 décrit, également, un dispositif de décontamination d'air comprenant une plaque de conduction de la lumière et une feuille de photocatalyseur à base d'oxyde de titane sur chaque face de la plaque (Fig.1A).

Le document EP-A-2047870 décrit un dispositif de décontamination d'air comportant un photocatalyseur et un tissu à base de carbone.

### Objet de l'invention

L'invention a pour but de proposer un dispositif de décontamination d'air remédiant aux inconvénients de l'art antérieur.

En particulier, l'invention a pour but de proposer un dispositif de décontamination d'air efficace, peu coûteux et inoffensif permettant d'éliminer les polluants de l'air c'est-à-dire les contaminants organiques, inorganiques mais également les microorganismes tels que les germes, par exemple, les bactéries, les virus ou les spores.

Selon l'invention, ce but est atteint par les revendications indexées.

En particulier, ce but est atteint par le fait que le support est poreux et pourvu d'une première couche interne comportant le composé métallique solide, d'au moins une seconde couche interne, lesdites première et seconde couches internes étant disposées entre deux couches externes comportant le photocatalyseur et par le fait que lesdits moyens de circulation créent un mouvement d'air le long du support et un écoulement d'air léchant et traversant le support.

Selon l'invention, ce but est également atteint par le fait que le composé métallique solide est un composé à base de cuivre métallique.

Selon un mode de réalisation préférentiel, le photocatalyseur est à base de dioxyde de titane.

D'autres caractéristiques techniques peuvent être utilisées isolément ou en combinaison :
- Le dispositif est doté d'un système déflecteur à la surface du support, ledit système déflecteur comportant le composé métallique solide ;
- Une source lumineuse est une source à rayonnement UV-A.

### Description sommaire des dessins

D'autres avantages et caractéristiques ressortiront plus clairement de la description qui va suivre des modes particuliers de réalisation de l'invention donnés à titre d'exemples non limitatifs et représentés aux dessins annexés, dans lesquels :
- La figure 1 représente une vue ouverte de dessus d'un dispositif de décontamination d'air selon un premier mode de réalisation particulier de l'invention.
- La figure 2 représente, schématiquement et en coupe selon AA, le dispositif selon la figure 1.
- La figure 3 représente, schématiquement et en coupe selon BB, le dispositif selon la figure 1.
- La figure 4 représente, schématiquement et en coupe, un support du dispositif selon la figure 1.
- La figure 5 représente, schématiquement et en coupe selon AA, une variante du dispositif selon la figure 1.
- La figure 6 représente, schématiquement et en coupe selon AA, un second mode de réalisation particulier de l'invention.
- La figure 7 représente, schématiquement et en coupe selon BB, le second mode de réalisation particulier de l'invention.
- La figure 8 représente, schématiquement et en perspective, un support du dispositif selon les figures 6 et 7.

### Description des modes de réalisation particuliers de l'invention

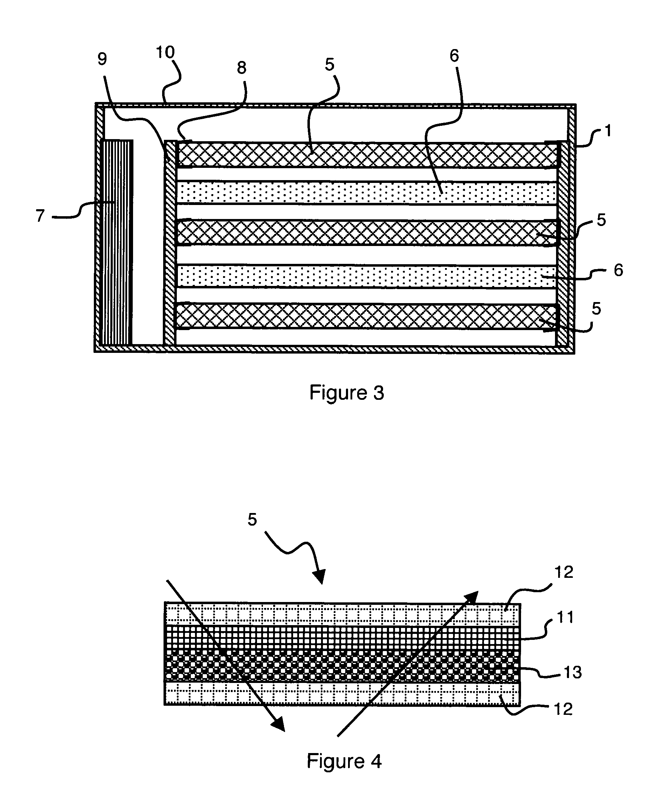

Selon un premier mode de réalisation particulier représenté aux figures 1 à 3, le dispositif de décontamination d'air, comporte une enceinte de photocatalyse 1 à l'intérieur de laquelle se déroulent les réactions de photocatalyse, responsables de la dégradation des contaminants de l'air. Des moyens de circulation d'air 2 dirigent l'air à décontaminer à l'intérieur de l'enceinte 1 par au moins un orifice d'entrée 3 et l'expulsent à l'extérieur par au moins un orifice de sortie 4 (figure 2). Les moyens de circulation d'air 2 sont, par exemple, un ventilateur et un système d'aspiration respectivement en entrée 3 et en sortie 4 de l'enceinte 1. L'enceinte 1 renferme au moins un support 5 comportant un photocatalyseur, au moins une source lumineuse 6 et une source d'alimentation électrique 7 alimentant en courant les moyens de circulation d'air 2 et la source lumineuse 6. Le support 5 est disposé parallèlement à la direction d'écoulement de l'air entrant (figure 2). Dans cette conformation, la résistance à l'air est faible et l'écoulement laminaire. Chaque extrémité latérale du support 5 (extrémités droite et gauche sur la figure 3) peut être renforcée, par exemple, par une tôle de renfort 8 formant ainsi une zone de fixation destinée à être solidarisée à l'enceinte 1. Le support 5 peut être fixé soit directement aux parois de l'enceinte 1 soit, comme illustré à la figure 3, par l'intermédiaire de cloisons internes 9. Pour faciliter la maintenance du dispositif, l'enceinte 1 peut également comporter un capot 10 donnant accès à l'intérieur de l'enceinte 1, pour remplacer, par exemple, les consommables tels que les sources lumineuses 6 ou les supports 5 usagés. Les moyens de circulation 2 créent un écoulement d'air léchant le long d'au moins une face principale du support 5 (flèches ondulantes sur la figure 2).

Le support 5 est, avantageusement, poreux et l'écoulement d'air est alors également traversant (flèches obliques sur la figure 2).

De préférence, le dispositif est composé de deux supports 5 externes (en haut et en bas sur la figure 2) et d'au moins un support 5 interne agencé entre deux sources lumineuses 6 et disposé entre les deux supports 5 externes. La source lumineuse 6 peut être constituée d'une ou plusieurs lampes, de préférence tubulaires (figure 3). Avantageusement, les supports 5 et les sources lumineuses 6 sont sensiblement parallèles entre eux.

La source lumineuse 6 est, avantageusement, une source à rayonnement ultraviolet UV-A, de longueur d'onde comprise entre 320 et 400 nm. La longueur d'onde préférée est égale à 365 nm. Les rayonnements UV-A présentent l'avantage d'être parmi les ultraviolets les moins nocifs pour l'être humain, notamment, comparativement aux ultraviolets à courte longueur d'onde (inférieure à 280 nm) tels que les UV-C connus pour leur nocivité.

Les sources lumineuses 6 illuminent au moins une face principale de chaque support 5 afin d'activer le photocatalyseur, de préférence, à base de dioxyde de titane (TiO₂), avantageusement du TiO₂ anatase. Comme représenté à la figure 3, les sources lumineuses 6 ainsi que les supports 5 sont parallèles entre eux afin d'obtenir une exposition du photocatalyseur la plus efficace possible. A titre d'exemple, la source lumineuse 6 est positionnée à une distance du support 5 comprise entre 3 mm et 25mm afin d'obtenir une puissance de 3eV, nécessaire à l'activation des sites photocatalytiques de TiO₂. Les rayonnements UV-A provoquent des modifications électroniques au sein du photocatalyseur TiO₂, créant alors des radicaux oxygénés aptes à attaquer les contaminants absorbés sur le TiO₂. Le photocatalyseur peut être fixé sur le support 5 selon tout procédé connu. Le support 5 est, avantageusement, un substrat poreux enduit ou imprégné par le photocatalyseur. À titre d'exemple, le support 5 poreux est à base de fibres cellulosiques, fibres de verre ou fibres non tissées, de préférence, synthétiques.

Par ailleurs, le dispositif comporte un composé métallique solide choisi parmi un métal germicide, bactériostatique ou antifongique, de préférence, un composé à base de cuivre métallique. En effet, le cuivre possède des propriétés germicide, bactériostatique et antifongique naturelles qui sont alors utilisées pour tuer les germes et spores, ou empêcher leur prolifération. Le composé métallique solide peut également être constitué de plusieurs métaux ayant des propriétés germicide, bactériostatique ou antifongique, par exemple, un composé à base de cuivre et d'argent métallique. Comme pour le support 5, le composé métallique solide peut être fixé sur un substrat selon tout procédé connu, avantageusement, sur un substrat poreux enduit ou imprégné par du cuivre métallique.

Préférentiellement, le composé métallique solide est associé au support 5 de sorte que les moyens de circulation 2 créent un mouvement d'air le long du support 5 et un écoulement d'air léchant et traversant le support 5, pour la mise en contact de l'air avec le composé métallique solide. Cette mise en contact ainsi que la présence d'oxygène dans l'air sont les principales conditions pour déclencher la réaction d'oxydation, responsable de la destruction des germes et autres microorganismes.

Comme représenté à la figure 4, le support 5 peut, avantageusement, être constitué d'une succession de couches poreuses comportant le photocatalyseur, le composé métallique solide mais également un absorbant, par exemple, un composé à base de carbone. Préférentiellement, le support 5 est poreux et formé par un empilement de couches contiguës (figure 4). Le support 5 est, avantageusement, pourvu d'une première couche interne 11 comportant le composé métallique solide, d'au moins une seconde couche interne (13) destinée à absorber les contaminants ou polluants. Les première et seconde couches internes, respectivement 11 et 13, sont disposées entre deux couches externes 12 comportant le photocatalyseur. Les moyens de circulation 2 créent un mouvement d'air léchant et traversant le support 5 pour la mise en contact de l'air avec le composé métallique solide. Ainsi, l'écoulement d'air arrivant à la surface libre des couches externes 12 traverse l'ensemble des couches poreuses, notamment, les première et seconde couches internes, respectivement, 11 et 13 et les couches externes 12 (flèches à la figure 4).

Une plaque de cuivre métallique, percée de micro-orifices, peut être utilisée pour constituer la première couche interne 11. On entend par micro-orifice un orifice ayant une taille ou un diamètre moyen adapté afin de favoriser la mise en contact de l'air avec le composé métallique tout en laissant passer l'air assurant ainsi un écoulement d'air traversant. Les micro-orifices ont, avantageusement, un diamètre moyen compris entre 0,05mm et 3mm, préférentiellement entre 0,1 mm et 2mm. Alternativement, la première couche interne 11 peut être constituée d'un tissu imprégné de cuivre métallique ayant un tressage adapté pour assurer l'écoulement de l'air et la mise en contact.

Selon un mode de réalisation particulier représenté à la figure 4, le support 5 est constitué d'un empilement de quatre couches successives suivantes :
- une première couche externe 12,
- une première couche interne 11,
- une seconde couche interne 13 et,
- une seconde couche externe 12.

Selon ce mode de réalisation particulier, la première couche interne 11 est adjacente à la première couche externe 12 et à la seconde couche interne 13. La seconde couche interne 13 est adjacente à la première couche interne 11 et à la seconde couche externe 12.

La seconde couche interne 13 est, avantageusement, constituée d'une couche de carbone structuré, par exemple, sous forme de microbilles. La couche de carbone structuré 13 permet d'optimiser la réaction photocatalytique en régulant l'apport en contaminants vers les sites photocatalytiques de TiO₂. En effet, en présence d'un excès de contaminants, la couche de carbone structuré 13 absorbe les contaminants en excès et évite ainsi la saturation des sites photocatalytiques. À l'inverse, lorsque la concentration en contaminants est faible, la couche de carbone structuré 13 résorbe les contaminants piégés en son sein, alimentant ainsi en réactifs les sites photocatalytiques. Par ce phénomène d'absorption/résorption, la saturation des sites photocatalytiques est évitée et la réaction photocatalytique optimisée. Comme représenté par les flèches obliques à la figure 2, le support 5 étant poreux, une partie du mouvement d'air créé le long du support 5 traverse le support 5. Ce mouvement traversant met en contact l'air avec le composé métallique solide présent dans la première couche interne 11 du support 5.

Selon une variante non représentée, le support 5 est constitué de deux couches de carbone structuré 13, disposées de part et d'autre d'une première couche interne 11 poreuse, imprégnée par du cuivre métallique. Les deux couches de carbone structuré 13 sont agencées entre les deux couches externes 12 comportant le photocatalyseur. De préférence, les couches externes 12 sont imprégnées de TiO₂ anatase. Cette structure multicouche est particulièrement adaptée au support 5 interne (figures 2 et 3) dont les deux faces principales sont exposées aux sources lumineuses 6.

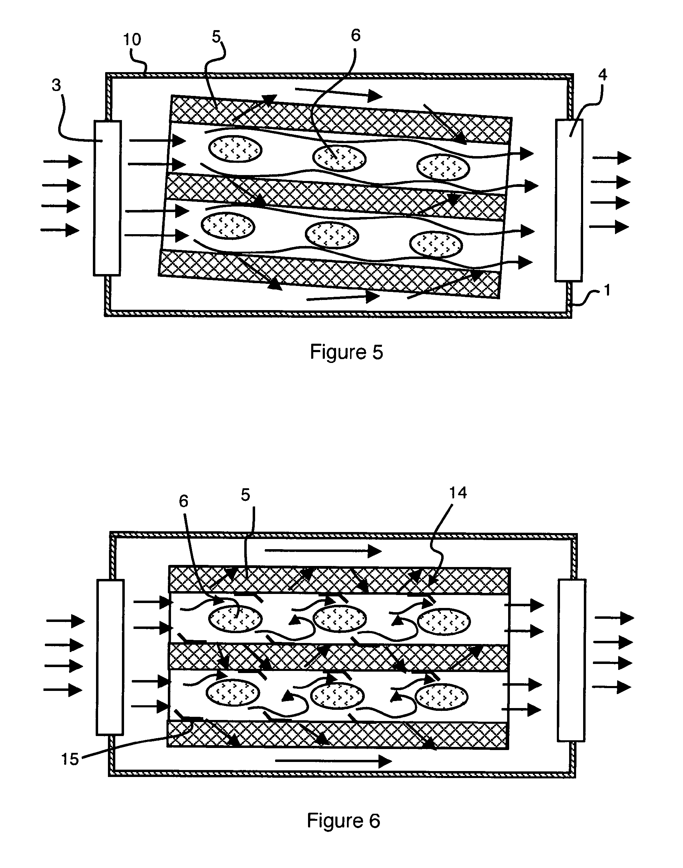

Selon une autre variante représentée à la figure 5, les supports 5, externes et internes, parallèles entre eux, sont inclinés d'un angle β compris ente 0° et 45° par rapport à la direction d'écoulement d'air entrant (flèches horizontales à gauche sur la figure 5). Cette inclinaison augmente l'effet traversant et, par conséquent, le temps de contact air/composé germicide, bactériostatique ou antifongique. Cette inclinaison favorise donc l'action germicide, bactériostatique ou antifongique du dispositif.

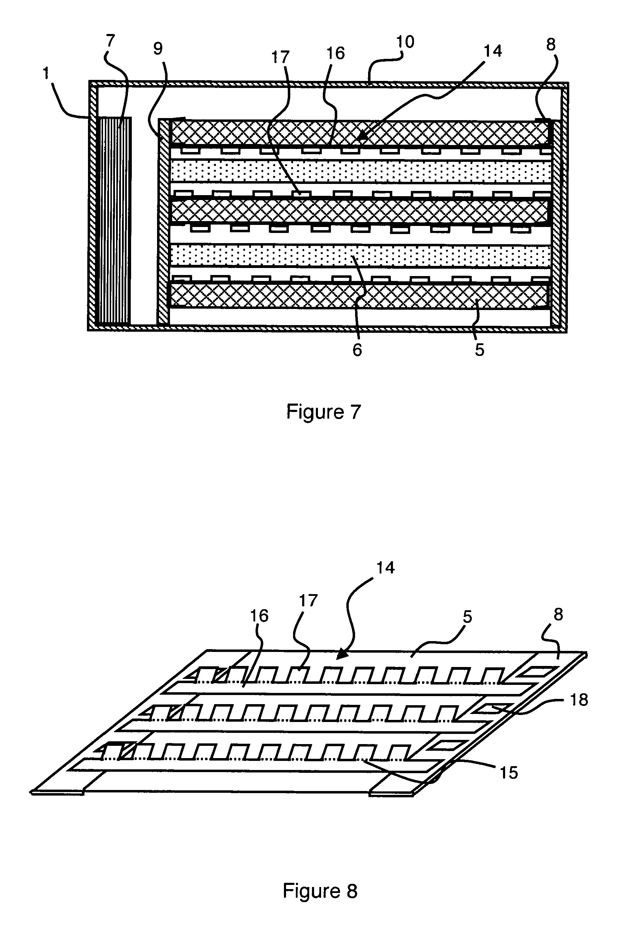

Selon un second mode de réalisation particulier représenté aux figures 6 et 7, le support (5) est doté, au moins sur une de ses surfaces, d'un système déflecteur (14) comportant le composé métallique solide. Le système déflecteur 14 est, avantageusement, constitué d'un ou plusieurs éléments formant saillies et répartis de façon homogène à la surface du support 5. Comme représenté à la figure 8, le système déflecteur 14 peut être réalisé à partir de lames dentées 15, inclinées par rapport à la surface du support 5 selon un angle α compris entre 0° et 45°. La lame dentée 15 est, avantageusement, réalisée à partir d'un substrat, de préférence, un matériau tissé imprégné de cuivre métallique. La lame dentée 15 est constituée d'une âme centrale 16 possédant sur un de ses bords longitudinaux des dents inclinées 17. Chaque extrémité de l'âme 16 est dotée d'un moyen d'accrochage 18 en vue de sa fixation au support 5. À titre d'exemple, le moyen d'accrochage 18 est constitué d'une languette passant à travers une lumière réalisée à l'extrémité du support 5 dans le prolongement de l'âme 16, de préférence, au niveau de la tôle de renfort 8 (figure 8). La languette est recourbée puis pressée afin d'enserrer le support 5 et la tôle de renfort 8.

Comme représenté à la figure 6, les lames dentées 15 dévient l'écoulement d'air léchant la surface du support 5 et créent des turbulences. Le brassage d'air ainsi obtenu, intensifie les mouvements d'air traversant (flèches obliques sur la figure 6). En combinant les mouvements d'air léchant et traversant, la réaction photocatalytique ne se fait plus uniquement en surface mais également dans la masse du support 5. Le nombre de passage de l'air près d'un site photocatalytique TiO₂ et par conséquent le temps de contact entre l'air et les sites photocatalytiques TiO₂ est plus important, ce qui améliore l'efficacité du dispositif. D'autre part, en réalisant un système déflecteur 14 à base de cuivre métallique, l'air entre en contact directement avec le composé métallique solide aux propriétés germicide, bactériostatique et fongique, ce qui favorise la destruction des microorganismes. Le système déflecteur 14 a, par conséquent, un effet double : un effet générateur de turbulences combiné à un effet germicide, bactériostatique et fongique.

Comme représenté aux figures 6 et 7, le système déflecteur 14 est disposé uniquement sur la face principale du support 5 faisant face à la source lumineuse 6. Ainsi, les supports 5 externes possèdent chacun un système déflecteur 14 uniquement sur une face principale tandis que le support 5 interne en possède deux, un sur chacune de ses deux faces principales.

Selon une variante non illustrée, le support 5 peut comporter à la fois un système déflecteur 14 et une première couche interne 11 à base de cuivre métallique. Le dispositif peut également comprendre des filtres à particules en entrée 3 et en sortie 4 du dispositif. Un pré-filtre, en entrée, peut être utilisé pour éliminer les particules risquant d'encrasser et/ou d'empoisonner le photocatalyseur. De même, un post-filtre, en sortie, peut permettre d'absorber les sous-produits de la réaction de photocatalyse tel que le dioxyde de carbone.

Le dispositif selon l'invention allie plusieurs avantages, un rendement de traitement élevé, une action germicide, bactériostatique et/ou fongique efficace ainsi qu'une conception simple et peu coûteuse.

Le dispositif selon l'invention permet, sans risque pour l'être humain, de décontaminer l'air ambiant non seulement en éliminant les principaux polluants organiques et inorganiques (fumées, solvants gazeux, etc...) mais également en combinant une action germicide, bactériostatique et/ou fongique. Ce dispositif est particulièrement adapté pour une utilisation dans les lieux publics, les hôpitaux ou dans les pièces nécessitant une vigilance particulière quant à la qualité de l'air, par exemple, dans les musées pour la sauvegarde des documents ou dans les salles blanches.

## Revendications

1. Dispositif de décontamination d'air par photocatalyse comportant :
- une enceinte de photocatalyse (1) pourvue d'au moins un orifice d'entrée (3) et un orifice de sortie (4),
- au moins une source lumineuse (6) pour activer la photocatalyse,
- au moins un support (5) comportant un photocatalyseur, ladite source lumineuse (6) illuminant au moins une surface du support (5),
- des moyens de circulation de l'air (2) à décontaminer en provenance de l'orifice d'entrée (3) vers l'orifice de sortie (4) de ladite l'enceinte (1),
- un composé métallique solide choisi parmi un métal germicide, bactériostatique ou antifongique,
dispositif **caractérisé en ce que** le support (5) est poreux et pourvu d'une première couche interne (11) comportant le composé métallique solide, d'au moins une seconde couche interne (13), lesdites première et seconde couches internes (11, 13) étant disposées entre deux couches externes (12) comportant le photocatalyseur et **en ce que** lesdits moyens de circulation (2) créent un mouvement d'air le long du support (5) et un écoulement d'air léchant et traversant le support (5).

2. Dispositif selon la revendication 1, **caractérisé en ce que** le composé métallique solide est un composé à base de cuivre métallique.

3. Dispositif selon l'une des revendications 1 et 2, **caractérisé en ce que** la seconde couche interne (13) est une couche de carbone.

4. Dispositif selon l'une quelconque des revendications 1 à 3, **caractérisé en ce que** la première couche interne (11) est une plaque de cuivre métallique percée de micro-orifices.

5. Dispositif selon l'une quelconque des revendications 1 à 4, **caractérisé en ce que** le support (5) est doté, au moins sur une de ses surfaces, d'un système déflecteur (14) formant saillie à la surface du support (5) et comportant le composé métallique solide.

6. Dispositif selon la revendication 5, **caractérisé en ce que** le système déflecteur (14) comporte une lame dentée inclinée (15) par rapport à la surface du support (5) selon un angle α compris entre 0° et 45°.

7. Dispositif selon l'une quelconque des revendications 1 à 6, **caractérisé en ce que** le support (5) est incliné d'un angle β compris entre 0° et 45° par rapport à la direction d'écoulement d'air entrant.

8. Dispositif selon l'une quelconque des revendications 1 à 7, **caractérisé en ce que** le photocatalyseur est à base de dioxyde de titane.

9. Dispositif selon l'une quelconque des revendications 1 à 8, **caractérisé en ce que** le dispositif est composé de deux supports (5) externes et d'au moins un support (5) interne agencé entre deux sources lumineuses (6), lesdits supports (5) et sources lumineuses (6) étant parallèles entre eux.

10. Dispositif selon l'une quelconque des revendications 1 à 9, **caractérisé en ce que** la source lumineuse (6) est une source à rayonnement UVA.
